# EUROPEAN PATENT APPLICATION

(11) **EP 4 623 950 A1**
(43) Date of publication of application: **01.10.2025**
(21) Application number: 24166341.8
(22) Date of filing: 26.03.2024
(51) Int. Cl.: A61M 5/142, A61M 5/145, A61M 5/24

(54) **INJECTION DEVICE WITH RELEASE LINER AND STRENGTHENING SHEET**

(71) Applicant: Ypsomed AG, 3401 Burgdorf (CH)
(72) Inventor: Dreisbach, Olaf, 2605 Sonceboz-Sombeval (CH); Indermaur, Martin, 3007 Bern (CH); Gartmann, Sandro, 3084 Wabern (CH)

(57) **Abstract**

The present application relates to an injection device comprising a housing with an adhesive layer on at least one side surface of the housing as well as a release liner (20) at least partially covering the adhesive layer. The release liner comprises a sheet of material with a first surface contacting the adhesive layer, as well as at least one aperture (32) defining a passage through the sheet of material. The release liner is covered by a strengthening sheet (40) adhesively connected to a second surface of the sheet of material and covering the at least one aperture. The strengthening sheet includes at least one area which forms a elongate recess (43) relative to the release liner, oriented towards the at least one aperture.

## Description

### Technical Field

The invention relates to an injection device with a release liner covering an adhesive layer arranged on a housing of the injection device. The release liner comprises a strengthening sheet which reinforces the release liner at least partially. The strengthening sheet is configured such as to reduce the occurrence of wrinkles and pleats when applying the strengthening sheet on the release liner.

### Background Art

Injection devices are used for the subcutaneous delivery of liquid medicaments to a patient. Such injection devices are often pen-shaped, having a long axis and are called injection pens. The injection pens comprise a housing, which can hold a dose setting and dose delivery mechanism. The medicament is preferably present in a cartridge or in a prefilled syringe. A cartridge is normally attached to the housing of the injection pen using a cartridge holder. The user sets a dose of medicament to be administered which is subsequently delivered from the cartridge. Such injection pens are used to deliver separate injections and not intended for continuous delivery of a medicament. The needle is attached to the injection pen each time before use and the needle penetrates a septum that is attached to the cartridge.

Another type of injection devices are infusion devices which deliver a medicament from the cartridge using a drive mechanism and a control mechanism that controls the advancement of a piston rod that abuts a moveable plunger present in the cartridge containing the medicament. The medicament is delivered to a patient via a fluid path and an external infusion set comprising a needle for subcutaneous delivery. With such infusion devices both continuous and temporary medicament delivery profiles can be programmed.

A patch device is an example of an infusion device that is attachable to the skin of a patient. Such patch devices do not need an external infusion set for delivery as the needle is directly contained in the patch device and may be inserted into the patient therefrom.

The injection devices comprise a dose setting mechanism, a delivery mechanism, a needle insertion and retraction mechanism or a needle shield protection system which is connected or connectable to the delivery mechanism. The delivery mechanism may be driven by a spring (compression spring, torsion spring, or leaf spring), an electromotor, pressurized gas or hydraulic systems and the like.

Usually, injection devices comprise a power source, such as a battery, which supplies energy to the injection or infusion device for executing tasks such as medicament delivery, establishing a connection between a fluid path and a cartridge, needle insertion, needle retraction, advancing and/or retracting a piston rod, signalling to a patient that the medicament administration is in progress and/or complete, signalling to a patient that the device can be removed, powering a processor unit in the device or establishing a wireless connection for data transmission to a remote device, for example to a mobile phone. Usually, several operations need to be performed in a certain sequence by an injection device for a correct operation. The sequence of operations may either be performed manually by a patient or automatically by an electronic control unit.

For a patch device, the needle must be inserted into a patient first, either using a steel needle (also called cannula) or a combination of a steel needle with a soft cannula. For the latter, after insertion, the steel needle has to be retracted to leave the soft cannula in the subcutaneous tissue of a patient The insertion of the steel needle or soft cannula into subcutaneous tissue of the patient is followed by delivery of the medicament. After medicament delivery, the needle, either a soft needle or a steel needle, is preferably retracted into the device, e.g. inside a housing thereof, before the patch device can be removed from the body. Alternatively, the needle is not retracted but a needle shield is extended from a housing of the device to protect the needle tip and prevent accidental pricking with the needle.

The medicament preferably is a liquid which is produced under sterile conditions and enclosed in a container to keep the medicament sterile. Such a container can be the above-mentioned cartridge or an ampoule, both of which are preferably made of glass. As an alternative, plastic containers may be used. The container comprises a barrel having two openings, one opening at the end of a neck portion and a second opening opposite to the neck portion. The opening at the neck portion is normally closed by a penetrable septum that is attached to the neck portion using a crimp. The opposite opening is closed by a plunger and the medicament is enclosed by the container between the septum and the plunger. During medicament delivery, the plunger is advanced in the container by a drive mechanism. The container is filled with the liquid medicament in a fill finish line, and either the plunger is inserted first into the container and the medicament is filled via the neck portion followed by closure using the septum crimped onto the neck portion, or the septum is attached first to the containers neck and the medicament is filled from the opposite opening and finally closed by the plunger. The fill finish is done in a sterile environment. The filled container is normally subjected to a visual inspection to ensure that no particulates are present in the liquid medicament.

The filled cartridge is assembled with an injection or infusion device, preferably a patch device, having a fluid path unit that is used for establishing the connection between the needle and the container.

The fluid path unit comprises a containment for housing a fluid path in the interior. The fluid path may comprise a spike that can pierce through the septum of the container, a tubing for fluid transfer connecting the spike to the hollow needle (which is either a soft needle and/or a steel needle) intended for penetrating the skin of a patient. Preferably, prior to a medicament delivery, there is no connection between the container and the fluid path. A connection there between is preferably established prior to medicament delivery by penetration of the septum with the spike. An interior of a containment for the fluid path unit is sterilized during manufacturing and remains in a sterile condition prior to a medicament delivery. The fluid path and the container can be assembled in a sterile environment which may be cumbersome (for example in view of the visual inspection) and expensive or it is assembled in a non-sterile environment such as a clean room. The device comprising the assembly of the fluid path and container can be contained in a sterile packaging prior to medicament delivery, the sterile packaging being removed before medicament delivery.

Patch devices are usually attached to skin of a patient prior to their use by means of an adhesive layer provided on the patch device. The adhesive layer is thereby protected by a release liner which is removed only before the patch device is attached to the skin of a patient. Often, the release liner is attached to at least one sterile barrier film inside of the patch device, such that the at least one sterile barrier film is removed simultaneously with the release liner. Removal of the release liner is performed by a patient by pulling off the release liner from the patch device. In order to strengthen the release liner in certain areas, especially in areas thereof which are attached to a sterile barrier film, the release liner may be provided with a strengthening sheet which is adhesively connected to the release liner as well as to the sterile barrier film. Provision of such a strengthening film greatly reduces the occurrence of ripping of the release liner and tearing off of the sterile barrier film from the release liner.

US2020/0086044 A1 discloses a patch pump comprising a disposable and a reusable part, wherein the disposable part comprises an adhesive surface for releasable attachment to a patient's skin. The adhesive surface is covered by a protective film that must be removed prior to attachment to the skin.

WO 2019/175689 A1 discloses a patch pump for releasable attachment to a patient's skin comprising an adhesive surface which is initially covered by a release liner. The release liner is reinforced by a strengthening sheet or bonding patch adhesively attached to the outer surface of the release liner.

However, as in certain areas of the patch device the surface to which the strengthening sheet is applied may not be completely flat but may comprise a convex bulge which is e.g. the result of the presence of a component protruding out of the surface of the patch device or being arranged on the release liner, for example the aforementioned sterile barrier film. A strengthening sheet adhesively applied to the release liner may wrinkle or form pleats in those areas. However, wrinkles or pleats negatively impact the visual appearance of the patch device and may create the impression of a faulty or defective assembly.

### Summary of the invention

It is therefore an object of the invention to create an injection device with a release liner reinforced by a strengthening sheet, the strengthening sheet being adhesively attached to the release liner in a wrinkle and pleat free manner.

The solution of the invention is specified by the features of claim 1. According to the invention a medicament delivery device comprises a housing with an adhesive layer on a bottom surface of the housing and with a release liner at least partially covering the adhesive layer. The release liner comprises a sheet of material with a first surface contacting the adhesive layer, as well as at least one aperture defining a passage through the sheet of material. The release liner is covered by a strengthening sheet or bonding patch adhesively connected to a second surface of the sheet of material and optionally covering the at least one aperture. The strengthening sheet includes at least one area which forms a concave recess relative a circumference of the release liner, such that the edge of the strengthening sheet is curved away from the circumference of the release liner in at least two sections, resulting in an area of the sheet that has a greater distance from the circumference of the release liner than neighbouring areas. Said at least one area is adapted such that the concave recess is oriented towards or at least partly arranged above the aperture of the release liner.

By providing a concave recess in the vicinity of the aperture, a minor elevation of the strengthening sheet caused by elements slightly protruding through the aperture can be mitigated to the recess area and the strengthening sheet remains free of any wrinkles or folds. Said elements may include a strip fed through the aperture and affixed to the release liner in the vicinity of the aperture. A smooth surface of the strengthening sheet enhances the visual appearance of the injection device perceived by the user.

The term "medicament" includes any flowable medical formulation suitable for controlled administration through a means such as, for example, a cannula or a hollow needle. The medicament may be a liquid, a solution, a gel or a fine suspension containing one or more medically active compounds. A medicament can be a composition comprising a single active compound or a pre-mixed or co-formulated composition with more than one active compound present in a single container. The medicament may comprise drugs such as peptides (e.g., insulin, insulin-containing drugs, GLP-1 containing drugs or derived or analogous preparations), proteins and hormones, active compounds derived from -or harvested by- biological sources, active compounds based on hormones or genes, nutritional formulations, enzymes and other substances in both solid (suspended) or liquid form but also polysaccharides, vaccines, DNA, RNA, oligonucleotides, antibodies or parts of antibodies as well as basic, auxiliary and carrier substances.

The distal end of the injection device is the end thereof which is oriented towards a patient during the injection of the medicament. Concurrently, the proximal end of the injection device is the end thereof which faces away of the patient during the injection of the medicament. The same applies to the terms "distal direction" and "proximal direction", i.e. the direction towards the patient during medicament injection and the direction away of the patient during medicament injection, respectively.

As the injection device comprises an adhesive layer on at least one side surface of the housing, the injection device according to the present invention is mainly configured as a patch device which may be adhered to skin of a patient by means of the adhesive layer.

The housing may be of any suitable shape, but preferably has a generally cuboid shape, most preferably with rounded corners and/or rounded edges. The housing is preferably made of a polymer material. The housing preferably is made of at least two parts which are assembled. The housing encloses an open space.

The adhesive layer preferably is located on a main bottom surface of the housing. Preferably the adhesive layer covers at least 50%, more preferably at least 70% of the surface area of the at least one side surface. The adhesive layer may comprise a skin adhesive material for adhesion to skin. Hence, the injection device may be releasably affixed to skin of a patient by means of the adhesive layer. This allows to temporarily connect the injection device on an injection site on the patient, i.e. for several minutes up to several hours or even days. The adhesive layer preferably is directly applied to the at least one surface of the housing. Alternatively, a circuit layer comprising an electrically conductive sensor may be located between the at least one surface of the housing and the adhesive layer. The electrically conductive sensor preferably is a capacitive sensor. In this case, the circuit layer preferably is affixed to the at least one surface of the housing by means of second adhesive layer and the adhesive layer is subsequently applied on the circuit layer.

The release liner is arranged on the adhesive layer and contacts the latter with a first surface. Thereby, the adhesive layer is at least partially covered by the release liner. Preferably, the release liner is configured to entirely cover the adhesive layer. Thereby, the release liner protects the adhesive layer from prematurely adhering to surfaces prior to the use of the injection device, i.e. prior to the affixation of the injection device on an injection site on a patient's skin. Further, the release liner also protects the adhesive layer from dust which may stick to the adhesive layer hence reducing the adhesive capacity of the latter. The release liner preferably is made of at least one sheet of material, preferably of at least one sheet of polymeric material. The release liner may comprise a conductive layer, for example a silver ink layer, carbon black layer, covering and being part of a signalling sensor, for example the electrically conductive sensor of the circuit layer as described above.

The at least one aperture through the sheet of material of the release liner may be of any suitable shape and size. Preferably, the at least one aperture is round, oval, rectangular polygonal or in the form of a stadium. The release liner may comprise only one aperture, but preferably comprises more than one aperture, such as two, three, four, five or more apertures. The at least one aperture may be located within the surface of the release liner, hence forming a hole through the sheet of material which is completely encircled by the sheet of material, or it may be located on an edge of the sheet of material, thereby forming an indentation along the edge of the sheet of material of the release liner.

The strengthening sheet preferably is adhesively affixed to a second surface of the sheet of material of the release liner, the second surface being located opposite the first surface. In order to adhesively affix the strengthening sheet on the release liner, the strengthening sheet preferably comprises an adhesive on an inner side oriented towards the release liner, i.e. the side thereof which is intended to be arranged on the release liner. Alternatively, the strengthening sheet may be affixed to the release liner by any other suitable means, such as welding, riveting or the like.

The strengthening sheet may only partially cover the release liner, specifically cover the release liner only in at least the area comprising the at least one aperture. Preferably, however, the strengthening sheet substantially covers the entire release liner, i.e. covers at least 70% or more thereof. In a preferred embodiment, the strengthening sheet completely covers the release liner.

The strengthening sheet preferably is made from a polymeric material, a sheet of paper or a woven or non-woven textile material. If the strengthening sheet is made of a polymeric material, the polymeric material preferably is or comprises polyethylene terephthalate, polybutylene terephthalate, polyester, or polyolefin, such as high-density polyethylene or polypropylene.

The strengthening sheet serves as reinforcement of the release liner such as to prevent any ripping of the release liner when the latter is removed from the adhesive layer. Further, the strengthening sheet may serve to securely affix other elements, such as a sterile barrier foil to the release liner, as any further element may thereby be connected between the release liner and the strengthening sheet, thereby ensuring that any further element is securely removed from the injection device upon removal of the release liner. Lastly, provision of the strengthening sheet may close the at least one aperture, thereby providing a sterile barrier which prevents contamination of the at least one aperture and any element which is inserted through the at least one aperture.

Preferably, a cartridge holder is arranged within the open space of the housing. The cartridge holder is preferably configured to hold a cartridge. The cartridge preferably comprises a container in which a medicament is contained. The container preferably is made of glass, such as borosilicate glass, or of a polymer material. The container comprises a barrel having two openings, one opening at the end of a neck portion and a second opening opposite to the neck portion. The opening at the neck portion is preferably closed by a penetrable septum that is attached to the neck portion, preferably by a crimp. A sterile barrier foil may be arranged on the septum such as to avoid any contamination of the septum. The opposite opening of the barrel is preferably closed by a plunger which is movable within the barrel in a dispensing direction. The medicament is enclosed by the container between the septum and the plunger. The cartridge preferably further comprises an encasing protecting the container and being provided with means for releasably coupling the cartridge with the cartridge holder.

Within the open space, the housing of the injection device preferably comprises a fluid path. The fluid path preferably comprises a spike for penetrating the septum of a cartridge held in the cartridge holder, as well as a tube connected to the spike with a first end thereof. A second end of the tube may be connected to a hollow needle or to a coupling for connecting the tube with a cannula. The fluid path hence enables to establish a fluid connection between the container and the hollow needle or cannula prior to the injection of the medicament by penetration of the septum by the spike. Therefore, the spike is preferably located next to the cartridge holder within the open space of the housing. Preferably, the fluid path is in a containment within the housing. The containment may either be delimited from the remainder of the open space by wall elements or by an inner housing. The inner housing preferably is a separate unit comprising the fluid path which is inserted into the open space of the housing during manufacture of the injection device.

The containment preferably comprises a first opening which is located towards the cartridge holder, the latter being configured such that when a cartridge is held in the cartridge holder, the septum will face or abut the first opening. The first opening preferably is covered by a sterile barrier foil in order to avoid any contamination of the fluid path prior to the use of the injection device, i.e. prior to the injection of the medicament. The fluid path is preferably sterilized while the sterile barrier foil is attached to the first opening. For example, the sterile barrier foil is made of a porous membrane such as Tyvek, and the containment is sterilized using gas plasma or ethylene oxide gas sterilization. Alternatively, radiation sterilization techniques may be used such as gamma-radiation, e-beam or X-rays.

The spike is preferably linearly movable towards the septum of the container of a cartridge held in the cartridge holder in order to pierce the septum to establish a fluid connection between the container and the fluid path. The spike may be manually movable by a patient, for example by means of a slider or rotary knob kinematically coupled to the spike. However, preferably, the spike is movable towards the septum by means of a spike drive. The spike drive may comprise a pre-tensioned spring, such as a torsion spring, helical spring, or at least one pre tensioned elastic element. Alternatively, the spike drive may comprise an electromechanical drive, a pneumatic or hydraulic drive or the like.

The housing preferably further comprises a drive mechanism for moving a piston of a container of a cartridge and an electronic control unit for controlling the device. The drive mechanism preferably comprises at least one electric motor which linearly drives a drive element, preferably by means of a gearing. The drive element is thereby configured to abut on the piston in order to move the latter within the barrel of the container in a dispensing direction, such as to push the medicament out of the first opening of the barrel.

The electronic control unit preferably comprises at least one microcontroller or processor, a printed circuit board as well as a source of electrical energy, such as a battery. The electronic control unit is configured to control the drive element to inject a correct amount of the medicament according to a pre-programmed or programmable dosage regimen.

The recess preferably has an elongated shape which is tapered a direction from a boundary of the sheet towards the at least one aperture in the release liner. The width of the recess decreases continuously in the vicinity of the aperture.

Provision of this recess allows to reduce the occurrence of any wrinkles or pleats when affixing the strengthening sheet on the release liner, as any misalignment of the strengthening sheet may be compensated by moving the edges of the elongated recess closer together, hence stretching the strengthening sheet.

Preferably, the at least one elongated recess has the shape of a trapezoid. Alternatively, the at least one elongated recess may be of any other suitable shape, such as triangular, rectangular, rhomboid, oval, or polygonal.

Preferably, the injection device comprises at least one film or strip connected to a sterile barrier foil located within the housing, said at least one film passing through the at least one aperture and being connected to an inner side of the strengthening sheet which is oriented towards the release liner.

With this configuration, the at least one film is securely affixed to the strengthening sheet so that once the release liner is removed from the housing, the at least one film is entrained by the removal movement and hence also removed. As the at least one film is connected to a sterile barrier foil, this configuration allows the removal of the at least one barrier foil together with the removal of the release liner. Preferably, the at least one film is connected to the strengthening sheet by means of an adhesive, preferably the same adhesive used to affix the strengthening sheet to the release liner.

Preferably, the at least one film or strip is connected to the second side of the release liner with a first film surface and to the inner side of the strengthening sheet with a second film surface, such as to be sandwiched between the release liner and the strengthening sheet. Provision of the strengthening sheet on the release liner thereby ensures that the at least one film is not disconnected or ripped from the release liner upon removal of the latter.

The at least one aperture is preferably configured as an indentation or cut-out on a side edge of the release liner. This allows to introduce the film or strip connected to the sterile barrier foil into the aperture by lateral insertion or displacement of the film into the indentation. In this configuration, the recess in the strengthening sheet is preferably at least partially coincident with the indentation.

Preferably, a first film connected to a first sterile barrier foil is inserted through the aperture, the first film being connected at least to the second side of the release liner, the first film being further inserted through a passage of the housing and the first sterile barrier foil closing at least one opening of a fluid path located within the housing.

The passage provides an opening through a side wall of the housing, through which the first film or any other element, such as for example a second film, may be inserted. As such, the first film may hence be lead from the open space enclosed by the housing to an outside of the housing.

The fluid path preferably is configured as described above, i.e. comprising a spike as well as an opening, whereby the first sterile barrier foil is affixed on the opening in order to close it. By passing the film through the passage of the housing and through at least one aperture of the release liner it is thereby possible to create a connection which allows the removal of the first sterile barrier foil from the opening simultaneously with the removal of the release liner. The passage is preferably located on the housing to be substantially congruent with the at least one aperture. Preferably, the first film is further connected to the inner side of the strengthening sheet.

The first film preferably is made of the same material as the first sterile barrier foil. More preferably, the first film and the first sterile barrier foil are a unitary piece of material, the first sterile barrier foil constituting a part of the unitary piece of material which has been sterilized and closes the sterilized fluid path. In this case, the first film is the remaining part of the unitary piece of material which does not close the sterilized fluid path. Preferably the first sterile barrier foil and preferably also the first film is made of a several non-woven porous polyethylene or polypropylene or paper (cellulose) films that are laminated together. Further preferably, the first sterile barrier foil and preferably also the first film is made of a fleece of polyethylene, such as Tyvek^{®} (as manufactured by DuPont de Nemours). The first sterile barrier foil and preferably also the first film is preferably coated to enhance or control the adhesive properties thereof.

Alternatively, the first film and the first sterile barrier foil may be made of different materials, i.e. the first film and the first sterile barrier foil are pieces of different materials which are connected together, for example by means of an adhesive, by means of welding or the like.

In order to protect the fluid path from contamination, the first sterile barrier foil is preferably attached circumferentially around the opening in a releasable manner, e.g. by means of a adhesive or the like.

Preferably, the injection device comprises a container held within the housing, said container containing a medicament and including at least one opening, the at least one opening being closed by a septum, a second sterile barrier foil being arranged on the septum, the injection device further comprising a second film connected to the second sterile barrier foil, the second film being inserted through the at least one aperture of the release liner and through a passage of the housing, the second film being connected at least to the second side of the release liner.

With this configuration, the second sterile barrier foil protecting the septum from contamination may be removed at the same time the release liner is removed. The container preferably is arranged within a cartridge as described above.

The second film preferably is made of the same material as the second sterile barrier foil. More preferably, the second film and the second sterile barrier foil are a unitary piece of material, the second sterile barrier foil constituting a part of the unitary piece of material which has been sterilized and is connected to the sterilized septum. In this case, the second film is the remaining part of the unitary piece of material which is not connected with the septum. Preferably the second sterile barrier foil and preferably also the second film is made of a fleece of polyethylene, such as Tyvek^{®} (as manufactured by DuPont de Nemours). Alternatively, the second film and the second sterile barrier foil may be made of different materials, i.e. the second film and the second sterile barrier foil are pieces of different materials which are connected, for example by means of an adhesive, by means of welding or the like.

Preferably, if the injection device comprises a first film and a second film, both films are inserted through the same aperture of the release liner and through the same passage of the housing. Alternatively, however, the first film is inserted through a first aperture of the release liner and a first passage of the housing while the second film is inserted through a second aperture of the release liner and a second passage of the housing.

Preferably, the housing is configured to guide the first film and/or the second film within the open space thereof. For example, the housing may comprise passages for the first and/or second film, delimited by wall elements, preferably with smooth or rounded edges, pins or protrusions present in the open space of housing or the like. This allows for a smooth removal of the first film and/or second film and the first sterile barrier foil and/or second sterile barrier foil, respectively, connected thereto.

Preferably, the injection device comprises a passage for a hollow needle, the hollow needle being configured to be moved from a first position, in which a tip of the hollow needle is located inside the housing, to a second position, in which the tip of the hollow needle is located outside of the housing, the passage being covered by a third sterile barrier foil, wherein the at least one aperture of the release liner is located on the opening and the third sterile barrier foil is adhesively attached to the inner side of the strengthening sheet.

As the third sterile barrier foil is connected to the strengthening sheet, the third sterile barrier foil is removed at the same time the release liner and strengthening sheet are removed, thus opening the passage.

Therefore, once the injection device is affixed to the patient by means of the adhesive layer, the hollow needle may be linearly moved through the opening in order to prick the patient prior to medicament injection. In order to linearly move the hollow needle, the injection device preferably comprises a needle insertion and retraction mechanism which is kinematically coupled to the hollow needle. The needle insertion and retraction mechanism may comprise at least one pre-tensioned spring, such as a torsion spring, helical spring, or at least one pre tensioned elastic element. Alternatively, the needle insertion and retraction mechanism may comprise an electromechanical drive, a pneumatic or hydraulic drive or the like.

Preferably, the injection device comprises a reusable drive module which is releasably coupled to the housing on a connection interface of the housing, the connection interface comprising at least one first mechanical connection means which is releasably connectable with at least one second mechanical connection means of the reusable drive module.

In this embodiment, the drive mechanism for moving the piston of the container of the cartridge and the electronic control unit for controlling the injection device are located in the reusable drive module. Hence, the reusable drive module may be repeatedly used by decoupling from the housing and coupling it to a new housing. The housing itself with any component located therein or thereon is thus configured as disposable module. As the drive mechanism and the electronic control unit do not need to be discarded after all the medicament has been dispensed from the container, but the reusable drive module may simply be coupled with a new housing, i.e. a new disposable module, the amount of waste as well as the costs for a patient may be greatly reduced. Further, the impact on the environment is also greatly reduced, as no electronic and mechanical components are being discarded each time that the container is empty.

The connection interface is preferably arranged on a side surface of the housing, i.e. not a main surface thereof. The reusable drive module preferably comprises a second housing. The second housing is preferably sized and shaped to match the size and shape of the housing, such that the injection device has an appealing shape when the reusable drive module is connected to the housing.

Preferably, the reusable drive module is fitted with components that, in connection with a disposable module, can be coupled or connected to the reusable module such as to enable a metered dispensing of the medicament contained in the container. Preferably, the reusable drive module comprises a drive element, such as, for example, a piston rod, which is coupled to the drive mechanism and which may be moved outside of the second housing or which protrudes from the second housing such as to be brought into contact with the plunger of the container. Further, the reusable drive module preferably comprises at least one electric contact, such as a pin, spring plate or the like, which is brought into engagement with an electrical contact element arranged on the connection interface of the housing, so that electrical energy, e.g. from a battery of the reusable drive module, may be transmitted to components within the open space of the housing, such as, for example, a needle extension and retraction mechanism.

The reusable drive module should preferably be designed so that it can couple to multiple disposable modules sequentially.

Preferably, the reusable drive module comprises a volatile or non-volatile memory element and/or a data transmission device for exchanging data with an external control element such as, for example, a mobile telephone, a computer or a pump control app running on a mobile device.

The first mechanical connection means and the second mechanical connection means are preferably configured as bayonet coupling. Alternatively, the first and second mechanical connection means may be in the form of a releasable snap-fit connection, a force fit connection, for example a threaded hole and a wing screw, or of any other suitable type.

Preferably, the first and second mechanical connection means are configured such that a patient has to put the two components, the disposable module and the reusable module, in correct alignment relative to each other to establish a connection there between. In the case of a bayonet connection, the patient thereby has to turn or engage the two modules into one another and subsequently twist them relative to one another by a predetermined angle, until the two modules lock into one another. The first and second mechanical connection means may, for example, be configured to automatically lock or snap with or into one another after a relative rotation has been performed. Preferably, an electrical connection is thereby also established between the modules. Preferably, the first and second mechanical connection means are configured such that the two modules are pulled together when establishing the connection there between. Further preferably, the first and second mechanical connection means are designed such that a play-free connection may be established by a patient with as little force exertion as possible.

Preferably, an electrical connection between the disposable module and the reusable drive module is only established when the two modules are correctly connected with each other. For example, the electrical contacts on the reusable drive module and the disposable module can be dimensioned so that they do not yet provide an electrical connection, that is to say, for example, so that there is still a gap between them, when the disposable module and the reusable drive module have not yet been sufficiently pulled together or, for example, screwed together, to establish a secure connection.

For example, the injection device according to this embodiment may be configured as disclosed in US 2020/0086044 A1.

Preferably, the connection interface includes at least one interface opening, the at least one interface opening being closed by a fourth sterile barrier foil, the fourth sterile barrier foil being connected to a fourth film, the fourth film being inserted through a third aperture of the release liner.

Hence, the at least one interface opening may be closed in a sterile manner by means of the fourth sterile barrier foil prior to medicament injection. The fourth sterile barrier foil may be removed together with the release liner, as the fourth sterile barrier foil is connected to the release liner by means of the fourth film.

Other advantageous embodiments and combinations of features may be derived from the detailed description below and the entirety of the claims.

### Brief description of the drawings

The drawings used to explain the embodiments show:
- Fig. 1: a first exemplary embodiment of an injection device;
- Fig. 2: an exploded view of the injection device shown in Fig. 1;
- Fig. 3: an embodiment of a strengthening sheet used with an injection device according to the present invention;
- Fig. 4: a schematic representation of a further embodiment of an injection device according to the present invention with a reusable drive module;
- Fig. 5: an embodiment of a cartridge which may be used with an injection device according to the present invention;
- Fig. 6: a longitudinal section of the injection device according to Fig. 1;
- Figs. 7 - 10: the final assembly of the injection device as shown in Fig. 1.

In the figures, the same components are given the same reference symbols.

### Preferred embodiments

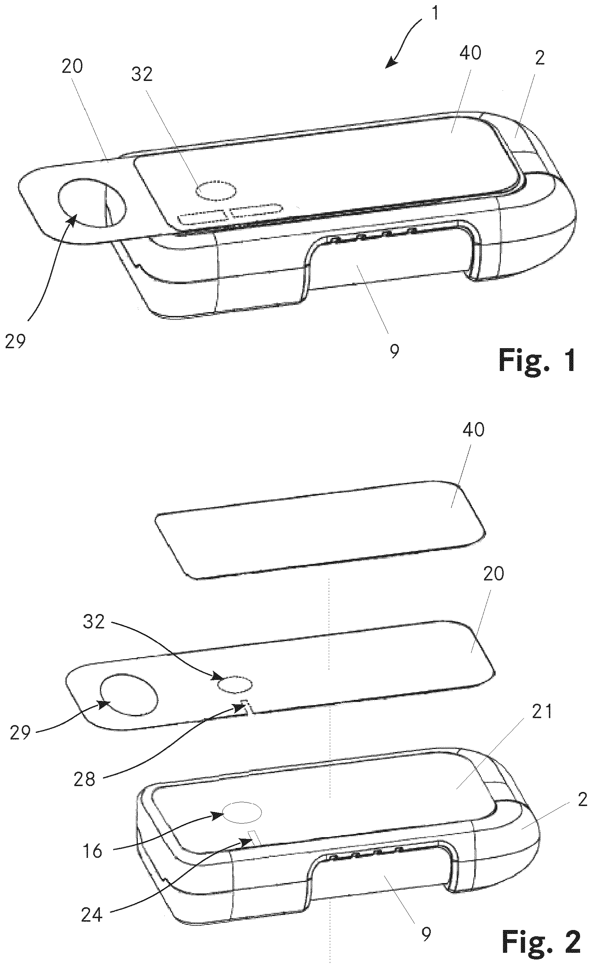

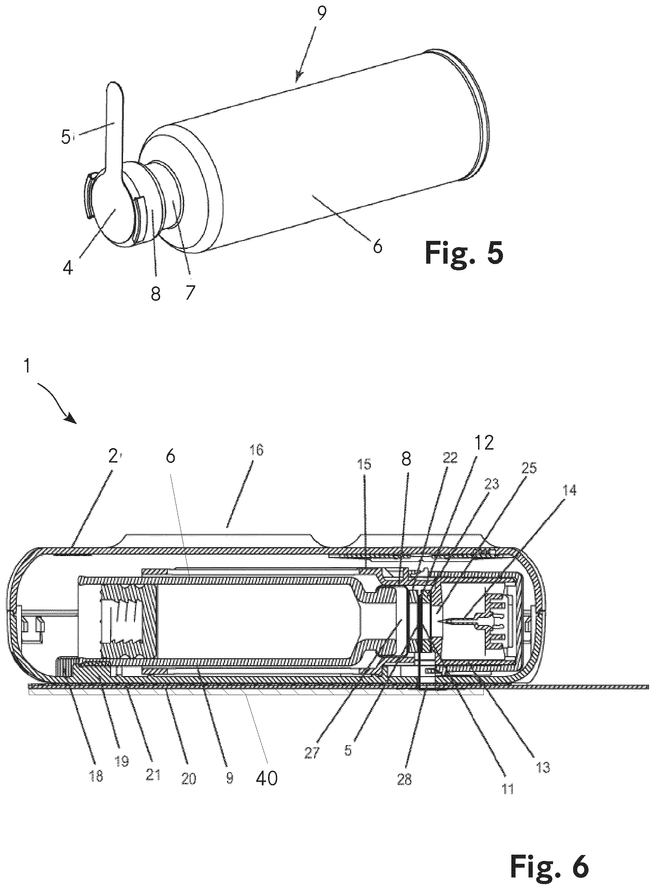

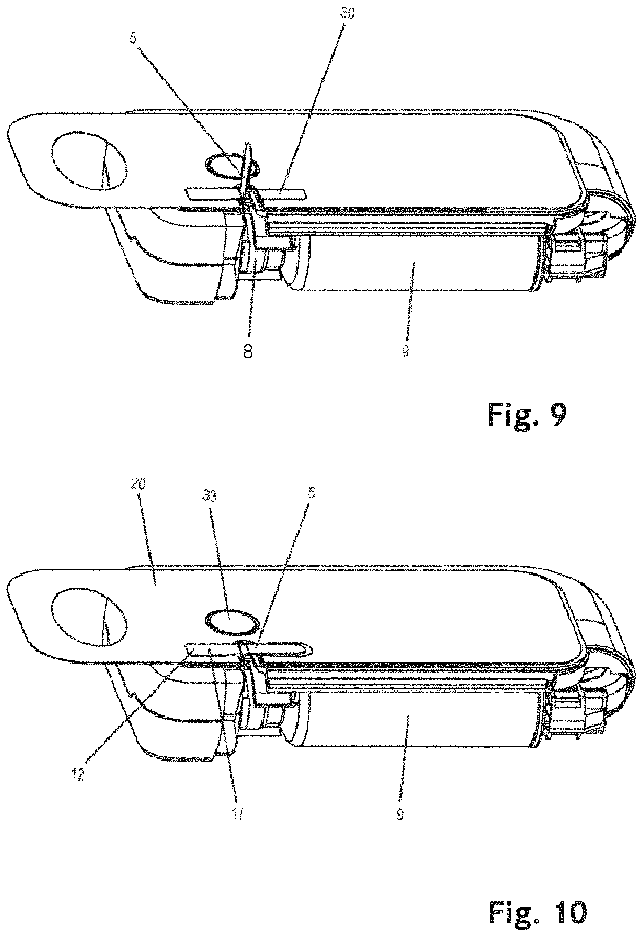

Fig. 1 shows a first exemplary embodiment of an injection device 1. The injection device 1 comprises a housing 2 which encloses an open space. A cartridge 9 including a container with a barrel 6 (see Figs. 5 and 6) comprising a medicament therein is inserted into the housing 2. An adhesive layer 21 (see Fig. 2) is arranged on a main bottom side of the housing 2. The injection device 1 may be removably attached to skin of a patient (not shown) by means of the adhesive layer 21. The adhesive layer 21 is protected by a release liner 20 which is arranged thereon. The release liner 20 covers the entire surface of the adhesive layer 21 in the embodiment shown. The release liner 20 protrudes from the housing 2 on one side in the embodiment shown, such as to facilitate the removal of the release liner 20 from the adhesive layer 21. Further, in the area protruding from the housing 2, the release liner 20 includes a cut-out 29 which allows for an easier grasping of the release liner 20 by a patient. The release liner 20 includes a first aperture 28 (see Fig. 2) which allows the passage of a first film 5 and of a second film 11 (see Fig. 10). The first film 5 and the second film 11 are bent and affixed on the release liner 20. The first aperture 28 is configured as an indentation on an edge of the release liner 20. Further, the release liner 20 includes a second aperture 32 which is circular in the embodiment shown. A third sterile barrier foil 33 is arranged within the second aperture 32.

A strengthening sheet 40 is affixed on the release liner 20 such as to at least partially cover the latter. Preferably, the strengthening sheet 40 is adhesively attached to the release liner 20.

Further, the injection device 1 further comprises a hollow needle (not shown) which is arranged within the open space of the housing 2 and which is movable from a first position, in which a tip of the hollow needle is located inside the open space, and a second position, in which the tip of the hollow needle protrudes from the housing 2. The hollow needle is arranged within the open space of the housing such that the tip of the needle may be moved through a second passage 16 (see Fig. 2) of the housing 2, the second passage 16 being arranged below the second aperture 32 of the release liner 20. in order to move the hollow needle, the injection device 1 comprises a needle insertion and retraction mechanism (not shown). Hence, when the injection device 1 is attached to skin of a patient by means of the adhesive layer 21, the hollow needle may be moved from the first position to the second position in order to prick the patient. Further, the injection device 1 also comprises drive means (not shown) in order to move a plunger 26 within the barrel 6 in order to discharge the medicament container in the barrel 6 via a fluid path (see Fig. 6) to the hollow needle. Lastly, the injection device 1 also includes an electronic control unit (not shown) arranged within the open space of the housing 2, which allows the control of the drive means as well as the needle insertion and retraction device.

Fig. 2 shows an explosion view of the injection device 1 shown in Fig. 1. In this figure, it may be seen that the release liner 20 is arranged on the adhesive layer 21 of the housing 2 such that the first aperture 28 is congruent with a first passage 24 of the housing 2 and the second aperture 32 is congruent with the second passage 16 of the housing 2.

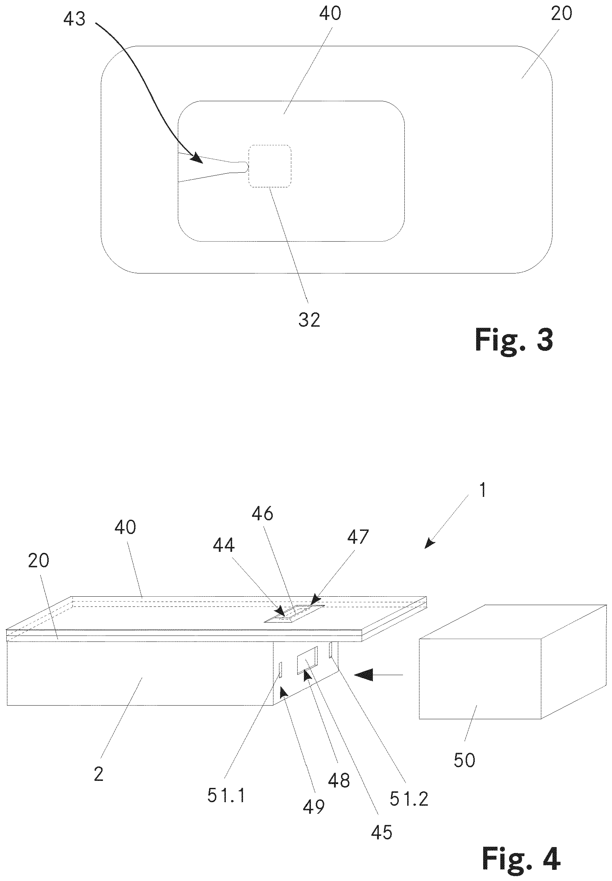

Fig. 3 shows an embodiment of a strengthening sheet 40 used with an injection device 1 according to the present invention. In this embodiment, the strengthening sheet 40 comprises a longitudinal recess 43 extending from a circumference of the sheet towards the aperture 32. The longitudinal recess 43 shown has a generally trapezoidal shape. Provision of the longitudinal recess 43 allows to further reduce the occurrence of wrinkles or pleats in the strengthening sheet 40 when affixing the strengthening sheet 40 on the release liner 20.

Fig. 5 shows an embodiment of a cartridge 9 which may be used with an injection device 1 according to the present invention. The cartridge 9 comprises a barrel 6, preferably made of glass or optionally made from a polymeric material such as polypropylene or cyclo-olefinic polymer (or copolymer). The barrel 6 is preferably cylindrically shaped having a narrowed neck 7 closed by a septum (see Fig. 6) that is connected to the cartridge 9 using a crimp 8. The crimp 8 has an opening surrounding the septum. A second sterile barrier film 4 is connected to the crimp 8 in front of the septum in order to protect the latter from any contamination. The second sterile barrier film 4 is connected to a flexible second film 5. In the embodiment shown, the second sterile barrier film 4 and the second film 5 are made of a single piece of material, the second sterile barrier film 4 constituting a sterilized portion of the single piece of material. The second film 5 spans away of the crimp 8 and is and available for attachment to the release liner 20 of the injection device 1, for example to the embodiment of the injector device 1 according to Fig. 1. The barrel 6 is filled with a medicament to be administered to a patient by means of the injection device 1.

Preferably, the second sterile barrier film 4 is a porous film that enables gas sterilization such as gas plasma (hydrogen peroxide) or ethylene oxide sterilization (ETO) to sterilize the septum of the cartridge.

Fig. 6 shows a longitudinal section of the injection device 1 according to Fig. 1. A cartridge 9, for example according to Fig. 5, is inserted into the housing 2 of the injection device 1. The injection device 1 may be attached to the skin of the patient using the adhesive layer 21 which is covered by the release liner 20. In the embodiment shown, a plunger 26 is movably arranged within the barrel 6 of the cartridge. The plunger 26 can move towards the septum 27 for expelling the medicament filled into the barrel 6 and being hold between the plunger 26 and the septum 27. The housing 2 encloses both the cartridge 9 and an encasing 13 for a fluid path. The fluid path comprises a spike 14, which is aligned parallel to a longitudinal axis of the cartridge 9, as well as an opening 25 which is arranged between the spike 14 and the cartridge 9. The opening 25 is closed by means of a first sterile barrier film 12. The first sterile barrier film protects the spike 14 from any contamination prior to the administration of the medicament by means of the injection device 1. As the second sterile barrier film 4 is affixed to the crimp 8 and arranged on the septum 27, the second sterile barrier film 4 and the first sterile barrier film 12 are arranged next to each other once the cartridge is inserted into the housing 2 of the injection device.

The cartridge 9 itself is positioned within a cartridge holder 15 arranged within housing 2, i.e. within an open space enclosed by the housing 2. The cartridge 9 is axially fixated in the housing 2 and biased towards the encasement 13 of the fluid path using a ratchet system. The ratchet system comprises a ratchet member 18 guided on an inside wall of the housing 2. The ratchet member 18 may be slid in the direction of a length axis of the housing 2 after the cartridge 9 has been inserted. Teeth of the ratchet member 18 thereby engage teeth of a counter ratchet member 19 that is axially fixed with respect to the housing 2. The ratchet member 18 engages a proximal rim of the barrel 6 of the cartridge 9 thereby enabling axial fixation of the cartridge 9 within the housing 2 and biasing the cartridge 9 towards the encasement 13 of the fluid path. Alternatively and instead of the ratchet member 18 the cartridge 9 may also be fixated using a ridge or fin on a cover closing the housing 2. During the closure of the housing 2 by means of the cover, the rip or fin is plastically deformed on the proximal rim of the cartridge thereby fixating the cartridge 9 within the housing 2.

The second sterile barrier film 4 is connected to the crimp 8 using a first member 22. The first member 22 may for example be an adhesive or a double-sided adhesive tape. Optionally the first member 22 may be a resilient member such as an O-ring, which is compressed once the cartridge 9 is fixated using the ratchet system. The second sterile barrier film 4 may also be directly heat-welded onto a distal end surface of the crimp 8. The second sterile barrier film 4 is connected to the crimp in such a way that this connection may be released by applying a force to an end of the second film 5, such as to peel-off the second sterile barrier film 4 from the crimp 8.

The opening 25 of the fluid path allows the spike 14 to at least partially move out of the encasement 13 such as to penetrate the septum 27. Movement of the spike 14 is enabled by a spike drive (not shown) arranged in the open space of the housing. The opening 25 is covered by the first sterile barrier film 12 which itself is connected to a first film 11. The first sterile barrier film 12 is attached to a wall of the encasement 13 and covers the opening 25, thereby preventing contamination of the fluid path, especially of the spike 14. Preferably, the first barrier film 12 is a porous film that enables gas sterilization such as gas plasma (hydrogen peroxide) or ethylene oxide sterilization (ETO) to sterilize the fluid path. The first sterile barrier film 12 is connected to the encasement 13 using a second member 23. The second member 23 may for example be an adhesive or a double-sided adhesive tape. Optionally the second member 23 may be a resilient member such as an O-ring which may be compressed once the cartridge 9 is fixated within the housing 2 using the ratchet system. The first sterile barrier film 12 may alternatively also be directly heat welded onto the wall of the encasement 13 in the area of the opening 25. The first sterile barrier film 12 is connected to the encasement 13 such that this connection may be released by applying a force to an end of the first film 11 to peel-off the first sterile barrier film 12 from the encasement 13 such that the opening 25 is opened for the spike 14 to move through.

The first film 11 and the second film 5 are guided through a passage 24 of the housing 2 such that the ends of the first film 11 and the second film 5 may be guided through an aperture 28 in the release liner 20. The ends of the first film 11 and of the second film 5 are connected to a second surface of the release liner 20. The strengthening sheet 40 is affixed onto the second side of the release liner 20 and covers the first film 11 and the second film 5.

By the connection of the first film 11 and the second film 5 to the release liner 20, both films 5, 11 are removed from the housing 2 when the release liner 20 is pulled off from the adhesive layer 21. As the first sterile barrier film 12 and the second sterile barrier film 4 are connected to the first film 11 and the second film 5, respectively, the first sterile barrier film 12 and the second sterile barrier film 4 are removed from the opening 25 and the crimp 3, respectively, upon removal of the release liner 20 from the adhesive layer 21. Optionally, a gap that may be present between the septum 27 and the encasement 13 after removal of the first sterile barrier film 12 and the second sterile barrier film 4 may be closed due to the resilience of the connecting members 22, 23 and/or by means of the ratchet system.

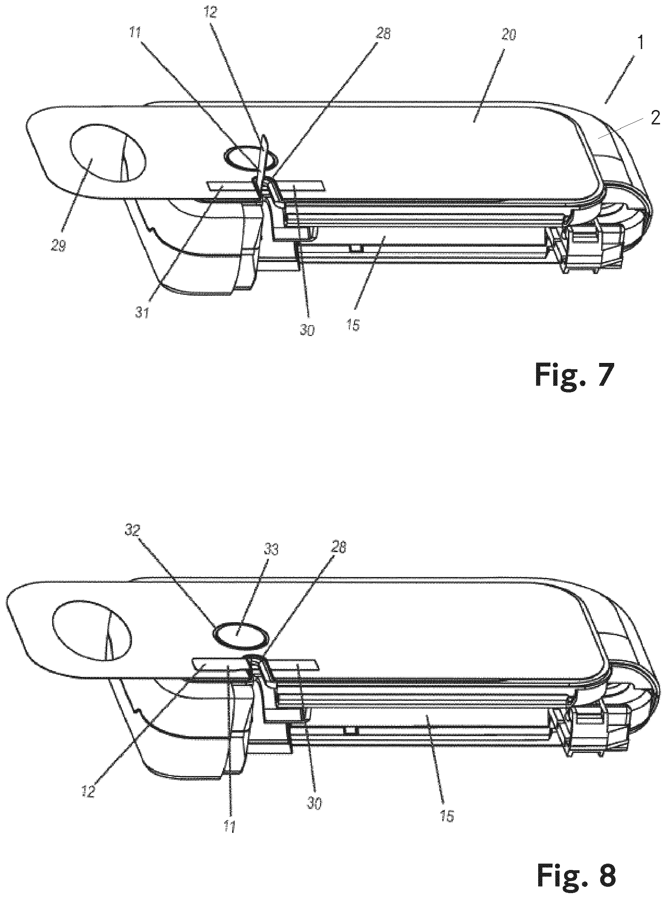

Figs. 7 to 10 show the final assembly of the injection device 1 as shown in Fig. 1. The injection device 1 is shown upside down with the release liner 20 covering the adhesive layer 21. The release liner 20 has a cut-out 29 acting as a handle to facilitate the removal of the release liner 20. The release liner 20 has a first aperture 28 which is in the form of a recess located at an edge of the release liner 20 to facilitate the insertion of the first film 11 from the side of the housing 2. The second side of the release liner 20, i.e. the side that is not contacting the adhesive layer 21, comprises a first adhesive spot 30 and a second adhesive spot 31. The adhesive spots 30, 31 may be a piece of adhesive tape.

As shown in Fig. 8, the end of the first film 11 is attached to the second side of the release liner 20 using the second adhesive spot 31. Thereby, the first film 11 is bent around an edge of the first passage 26. Alternatively, the second adhesive spot 31 may be present at the end of the first film 11. Other attachment means for the end of the first film 11 are, for example, heat welding or ultrasonic welding. The surfaces of the release liner 20 and of the first film 11 which need to be connected to each other may be roughnened, etched, blasted, heat treated or plasma treated to enhance the adhesive connection there between.

Once the end of the first film 11 has been connected to the release liner 20, the cartridge 9 is inserted sideways into the housing 2 and connected to the cartridge holder 15, as shown in Fig. 9. An end of the second film 5 is guided into the recess shaped first aperture 28 during cartridge 9 insertion and the first film 11 and the second film 5 may be arranged parallel to another and contact each other.

The end of the second film 5 is attached to the second side of the release liner 20 using the first adhesive spot 30, as shown in Fig. 10. The other options disclosed above for attaching the first film 11 to the second adhesive spot 31 equally apply to attachment of the second film 5 to the first adhesive sport 30. The sequence disclosed in Figs 7 to 10 may also be reversed, i.e. the cartridge 9 may be inserted and the second film 5 may be attached to the release liner 20 followed by the attachment of the first film 11 to the release liner.

The release liner 20 comprises a second aperture 32 that surrounds a third sterile barrier film 33. The third sterile barrier film 33 is removably connected to the housing 2 of the injection device 1. The third sterile barrier film 33 covers a second passage 16 of the housing 2 that is intended to allow a hollow needle to move from a first position, in which a tip of the hollow needle is within the open space of the housing 2, to a second position, in which the tip of the hollow needle protrudes from the second passage 16 in order to prick a patient. The hollow needle preferably moves perpendicular to the plane of the release liner 20. The third sterile barrier film 33 is preferably made from the same material as the first and second sterile barrier films 4, 12 and allows for gaseous sterilization techniques. The release liner 20 is preferably not directly connected to the third sterile barrier film 33 but surrounds it.

The strengthening sheet 40 is attached to the release liner 20 and thereby covers at least the end of the first film 1, the end of the second film 5, the third sterile barrier film 33 and the apertures 28, 32 of the release liner 20. The strengthening sheet 40 is preferably adhesively attached to the second side of the release liner 20, the first and second films 5, 11 and the third sterile barrier film 33. The strengthening sheet 40 strengthens the release liner 20 thereby preventing mechanical damage to the release liner 20 due to stress, when the first and second films 5, 11 are pulled out of the housing 2 (thereby releasing the connection between the second sterile barrier film 4 and the septum 27 as well as the connection between the first sterile barrier film 12 and the opening 25) and the third sterile barrier film 33 is removed from the housing 2. The final assembly of the injection device 1 with the strengthening sheet is shown in Fig. 1.

Fig. 4 schematically shows an alternative embodiment of an injection device 1 according to the present invention. In this embodiment, the injection device 1 comprises a housing 2 as well as a reusable drive module 50 which may be releasably coupled to the housing 2. The housing 2 may also be referred to as disposable module, since the housing 2 may be uncoupled from the reusable drive module 50 after the medicament has been completely dispensed from the container and the reusable drive module 50 may subsequently be coupled with a new housing 2, i.e. with a new disposable module. The housing 2 comprises a coupling interface 49 which allows the releasable coupling of the housing 2 with the reusable drive module 50. In order to enable this coupling, the coupling interface 49 comprises two first mechanical coupling elements 51.1, 51.2 which are configured as slots in the embodiment shown. The reusable drive module comprises matching second mechanical coupling elements, which are not visible in the perspective as shown in Fig. 4. Further, the coupling interface 49 includes an interface opening 48 through which an element of the reusable drive module 50 may be inserted into the open space of the housing 2, such as for example a piston rod of a drive mechanism. The interface opening 48 is closed by means of a fourth sterile barrier film 45 such as to protect the open space of the housing 2 from contamination. The fourth sterile barrier film 45 is connected to a fourth film 46 which is inserted through a third passage of the housing and a third aperture 44 of the release liner 20, the third aperture 44 being arranged on the third passage of the housing 2. A third area 47 of the strengthening sheet is arranged such as to cover the third aperture 44. The fourth film 46 is connected at least to the release liner 20 so that the fourth sterile barrier film 45 may be removed together with the removal of the release liner 20 such as to open the interface opening 48.

Preferably, the strengthening sheet is applied on the release liner by means of an applicator, preferably an applicator which holds the strengthening sheet by means of vacuum. The applicator is preferably configured to bring the strengthening sheet into contact with the release liner at an acute angle, preferably at an angle of between 5° and 45°, whereby the applicator is subsequently slid over the release liner at said angle. This may be accomplished by providing an appropriate rig which allows to slide the applicator across or along the release liner with the proper angulation. Preferably, the strengthening sheet includes an adhesive applied on an inner side of the strengthening sheet which is intended to be in contact with the second side of the release liner This allows to reduce the formation of air bubbles between the strengthening sheet and the release liner, which may degrade the visual appearance of the injection device.

A method of adhesively applying a strengthening sheet (40) to a release liner (20) covering an adhesive surface (21) of the medicament delivery device (1) may thus comprise the steps of
Non-adhesively attaching the strengthening sheet to a surface of an applicator using negative pressure, wherein the surface of the applicator has the same shape as the strengthening sheet and an adhesive surface of the strengthening sheet is facing away from the applicator;
Moving the surface with the strengthening sheet above the second surface of the release liner at an angle between 0° and 90°, preferably between 5° and 45°;
Moving the applicator towards the release liner wherein a first edge of the applicator makes contact with a side area of the release liner such that an edge of the strengthening sheet is attached to the release liner;
Moving the applicator towards the opposite side of the release liner, wherein the strengthening sheet is pressed against the liner by an edge of the applicator and attached to the release liner in a bubble-free manner.

## Claims

1. A medicament delivery device (1) for injection or infusion of a substance, comprising a housing (2) with an adhesive layer (21) on a surface of the housing as well as a release liner (20) at least partially covering the adhesive layer, the release liner comprising a sheet of material with a first surface contacting the adhesive layer, as well as an aperture (28, 32) defining a passage through the sheet of material, wherein the release liner is covered by a strengthening sheet (40) connected to a second surface of the sheet of material, **characterized in that** the strengthening sheet includes an area which forms a recess (43) oriented towards or at least partially arranged above the aperture of the release liner.

2. The medicament delivery device according to claim 1, **characterized in that** the recess has an elongated shape which is tapered towards the aperture.

3. The medicament delivery device according to claim 1, **characterized in that** the recess has at least in part the shape of a trapezoid with rounded corners, and with the shorter of its bases being located towards or above the aperture.

4. The medicament delivery device according to any of claims 1 to 3, **characterized in that** the medicament delivery device comprises a film connected to a sterile barrier foil located within the housing, said film passing through the aperture and being connected to an inner side of the strengthening sheet by means of an adhesive.

5. The medicament delivery device according to claim 4, **characterized in that** the film is connected to the second surface of the sheet of material of the release liner with a first film surface and to the inner side of the strengthening sheet with a second film surface.

6. The medicament delivery device according to any of claims 4 or 5, characterized that the at least one aperture is configured as an indentation on a side edge of the release liner.

7. The medicament delivery device according to any of claims 4 to 6, **characterized in that** a first film connected to a first sterile barrier foil is inserted through the aperture and through a passage of the housing, with the first sterile barrier foil closing at least one opening of a fluid path located within the housing.

8. The medicament delivery device according to any of claims 4 to 7, **characterized in that** the injection device comprises a container held within the housing, said container containing a medicament and including at least one opening, the at least one opening being closed by a septum, a second sterile barrier foil being arranged on the septum, the injection device further comprising a second film connected to the second sterile barrier foil, the second film being inserted through the at least one aperture and through a passage of the housing.

9. The injection device according to any of claims 1 to 8, **characterized in that** the injection device comprises a passage for a hollow needle, the hollow needle being configured to be moved from a first position, in which a tip of the hollow needle is located inside the housing, to a second position, in which the tip of the hollow needle is located outside of the housing, the passage being covered by a third sterile barrier foil, wherein the aperture of the release liner is located on the passage.

10. The medicament delivery device according to any of claims 1 to 9, **characterized in that** the injection device comprises a reusable drive module which is releasably coupled to the housing on a connection interface of the housing, the connection interface comprising at least one first mechanical connection means which is releasably connectable with at least one second mechanical connection means of the reusable drive module.

11. The medicament delivery device according to claim 10, **characterized in that** the connection interface includes at least one interface opening, the at least one interface opening being closed by a fourth sterile barrier foil, the fourth sterile barrier foil being connected to a fourth film, the fourth film being inserted through a third aperture of the release liner.
